# FASCICULE DE BREVET EUROPEEN

(11) **EP 1 001 801 B1**
(45) Date de publication et mention de la délivrance du brevet: **02.01.2002**
(21) Numéro de dépôt: 98941527.8
(22) Date de dépôt: 03.08.1998
(51) Int. Cl.: A61K 38/21, A61K 31/70

(54) **PRODUIT COMPRENANT AU MOINS UN ARN DOUBLE BRIN EN ASSOCIATION AVEC AU MOINS UN INTERFERON POUR LE TRAITEMENT DE L'HEPATITE VIRALE**
ZUSAMMENSETZUNG DIE MINDESTENS EIN DOPPEL STRANGIGES RNA UND MINDESTENS EIN INTERFERON ENTHÄLT, ZUR BEHANDLUNG VON VIRALE HEPATITIS
PRODUCT COMPRISING AT LEAST A DOUBLE STRANDED RNA COMBINED WITH AT LEAST AN INTERFERON FOR THE TREATMENT OF VIRAL HEPATITIS

(30) Priorité: 04.08.1997 FR 9709975; 26.08.1997 FR 9710644; 17.09.1997 FR 9711543
(43) Date de publication de la demande: 24.05.2000
(73) Titulaire: SOCIETE DE CONSEILS DE RECHERCHES ET D'APPLICATIONS SCIENTIFIQUES (S.C.R.A.S.), 75016 Paris (FR)
(72) Inventeur: DESCHAMPS DE PAILLETTE, Evelyne, F-75016 Paris (FR)
(74) Mandataire: Bourgouin, André
(86) Numéro de dépôt international: FR9801727
(87) Numéro de publication internationale: WO9907409

(56) Documents cités:
- EP-A- 0 213 921
- EP-A- 0 281 380
- EP-A- 0 286 224
- EP-A- 0 300 680
- EP-A- 0 306 347

## Description

La présente invention concerne l'utilisation, pour préparer un médicament destiné au traitement d'une hépatite virale, d'un produit comprenant au moins un ARN double brin (ARNdb) en association avec au moins un interféron, pour une utilisation thérapeutique étalée dans le temps. L'administration d'ARNdb a lieu avant celle de l'interféron.

La demanderesse a trouvé que l'association d'ARNdb avec l'interféron, procure un effet synergique inattendu dans le traitement des maladies virales, notamment le traitement d'une hépatite virale.

Par ARNdb, on entend de préférence l'acide polyadénylique complexé avec l'acide polyuridylique, aussi appelé poly(A)-poly(U) ou Poly-adenur®. D'autres ARNdb peuvent être utilisés pour l'invention, notamment un complexe de l'acide polyinosinique avec l'acide polycytidylique, également connu sous le nom de poly(I)-poly(C), ainsi que ces mêmes complexes modifiés par introduction d'acide uridylique dans la chaîne de l'acide polycytidylique, tel le produit Ampligen® de la société HEMISPHERx (pour une description de ces produits, se référer notamment à la demande de brevet européen EP 0 300 680). L'ARNdb utilisé peut être par exemple un mélange d'ARNdb tels que défini ci-dessus. De préférence, les ARNdb sont préparés selon le procédé décrit dans le brevet français No. 2 622 586.

Par agent anti-viral, on entend dans la présente demande aussi bien un agent agissant directement sur le virus, tel que par exemple la ribavirine ou la lamivudine, qu'un agent immunomodulateur, c'est-à-dire un agent qui diminue ou renforce les défenses immunitaires, tel que la ciclosporine ou un interféron. Les agents anti-viraux associés à l'ARNdb et à l'interféron pourront par exemple être choisis parmi un interféron tel que les interférons α, β et γ ou les interférons consensus, et en particulier un interféron α (IFN-α), d'autres lymphokines comme les interleukines, par exemple l'IL 6 ou l'IL 9, le ganciclovir, la coumermycine A1, la lamivudine, la ribavirine, la vidarabine, la didéoxyinosine (DDI), l'azathioprine, la prednisolone et la ciclosporine. De préférence, l'agent anti-viral associé à l'ARNdb sera un interféron.

Par interféron α, on entend l'un ou plusieurs des différents interférons α tels que par exemple les interférons α-2a, α-2b, α-2c, α-n₁, α-n₃, ou tout autre analogue possédant des propriétés immunologiques comparables. Par interféron consensus, on entend par exemple les interférons IFN-con1, IFN-con2 et IFN-con3 (ces interférons consensus sont notamment décrits dans le brevet américain US 5,372,808 ou la demande de brevet PCT WO 93/21229).

Par utilisation thérapeutique étalée dans le temps, on entend une administration de plusieurs principes actifs à des moments différents et notamment un mode d'administration selon lequel l'ensemble de l'administration de l'un des principes actifs est effectué avant que l'administration de l'autre ou des autres ne commence. On peut ainsi administrer l'un des principes actifs pendant plusieurs mois avant d'administrer l'autre ou les autres principes actifs. Il n'y a pas de traitement simultané dans ce cas.

Par maladie virale, on entend notamment une hépatite virale, et en particulier l'hépatite B ou l'hépatite C. Les hépatites virales traitées par le produit selon l'invention pourront être de type chronique ou aigu. De préférence, le produit de l'invention s'adresse aux hépatites chroniques.

L'invention concerne donc notamment l'utilisation, pour la préparation d'un médicament, d'un produit caractérisé en ce qu'il comprend :
i) un ARNdb en association avec
ii) un interféron
pour une utilisation thérapeutique étalée dans le temps dans le traitement d'une maladie virale. L'utilisation d'ARNdb a lieu avant celle de l'agent anti-viral.

De préférence, l'inteféron utilisé sera un interféron α.

Selon un aspect particulier de l'invention, le produit comprend au moins un ARNdb en association avec au moins un interféron, et est caractérisé en ce qu'il comprend également au moins un agent anti-viral agissant directement sur les virus pour une utilisation simultanée ou séparée avec le ou les interférons dans le traitement d'une maladie virale.

Selon une autre variante de l'invention, le produit, qui comprend au moins un ARNdb en association avec au moins un interféron, est caractérisé en ce qu'il comprend en outre au moins un autre agent anti-viral pour une utilisation thérapeutique étalée dans le temps et antérieure à celle de l'ARNdb dans le traitement d'une maladie virale. Parmi les autres agents anti-viraux utilisables pour cette variante particulière de l'invention, on peut citer d'autres lymphokines que les interférons comme les interleukines, par exemple l'IL 6 ou l'IL 9, le ganciclovir, la coumermycine A1, la lamivudine, la ribavirine, la vidarabine, la didéoxyinosine (DDI), l'azathioprine, la prednisolone et la ciclosporine.

Tout particulièrement, l'invention concerne un produit caractérisé en ce qu'il comprend :
i) un agent anti-viral en association avec
ii) un ARNdb,
et iii) un interféron éventuellement associé simultanément ou séparément à un agent anti-viral agissant directement sur les virus
pour une administration séparée dans le temps et effectuée dans l'ordre indiqué ci-dessus dans le traitement d'une maladie virale, en particulier d'une hépatite virale.

Différentes séquences d'administration de l'ARNdb et de l'agent anti-viral peuvent être envisagées. Selon un mode particulier de l'invention, l'ARNdb et l'antiviral ne sont pas administrés simultanément. De façon préférentielle, l'ARNdb est administré avant l'agent anti-viral associé. Le traitement par l'ARNdb est étalé de préférence pendant une période de 1 à 12 mois ou plus, par exemple 6 mois, et suivi par une administration sur une durée équivalente ou différente de l'agent anti-viral.

De préférence, la maladie virale traitée par les produits et compositions pharmaceutiques selon l'invention sera une hépatite virale, telle l'hépatite A, B, C ou G ou les hépatites "non A, non B, non C, non G", c'est-à-dire d'autre type que les hépatites A, B, C ou G. Les produits et compositions pharmaceutiques selon l'invention s'adresseront tout particulièrement au traitement de l'hépatite B ou l'hépatite C. Par ailleurs, les hépatites virales traitées par les produits et compositions pharmaceutiques selon l'invention peuvent être des hépatites virales aiguës ou chroniques et seront de préférence des hépatites virales chroniques. Les hépatites virales traitées par les produits et compositions pharmaceutiques selon l'invention seront de façon particulièrement préférentielle les hépatites chroniques B ou C.

Les compositions pharmaceutiques comprenant un produit selon l'invention peuvent être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un produit selon l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

Le mode d'administration d'un produit selon l'invention est choisi parmi les modes d'administration classiques. Ainsi, l'administration d'ARNdb peut par exemple se faire par voie topique, orale, parentérale, par injection intramusculaire ou intraveineuse, ou par voie sous-cutanée. De même, l'administration des agents anti-viraux peut se faire selon les mêmes voies. Pour chacun de ces composés, l'homme du métier choisira la méthode d'administration la plus appropriée.

La dose d'administration envisagée pour médicament selon l'invention est comprise entre 0,1 mg à 10 g par administration suivant le type de composé actif utilisé.

Pour l'ARNdb, notamment pour le poly(A)-poly(U), on pourra envisager une dose de préférence comprise entre 10 mg et 3 g par prise. Le produit peut être administré de façon journalière ou plusieurs fois par semaine. On peut par exemple administrer de 15 mg à 1,5 g, de préférence de l'ordre de 50 à 300 mg deux ou trois fois par semaine.

La dose d'interféron sera en principe celle couramment utilisée par l'homme du métier, et de préférence comprise entre 0,5 et 60 millions d'unités internationales par prise. Avec un IFN-α, la prise peut être par exemple de 1 à 50 millions d'unités, de préférence entre 1 et 10 et notamment entre 3 et 6 millions d'unités. Par ailleurs, l'administration peut être journalière ou effectuée plusieurs fois par semaine. On peut notamment effectuer deux ou trois administrations par semaine. Par exemple, on pourra choisir d'administrer entre 3 et 6 millions d'unités deux ou trois fois par semaine.

Selon une autre variante de l'invention, on pourra en outre faire précéder le traitement par l'ARNdb par un traitement par un agent anti-viral. Par exemple, on pourra administrer durant une première période un interféron, de la lamivudine ou de la ribavirine, durant une deuxième période de l'ARNdb, et enfin durant une troisième période un interféron éventuellement associé à au moins un autre agent anti-viral tel la ribavirine ou la lamivudine. De préférence, l'interféron employé pour le traitement sera un IFN-α.

Pour choisir les modes et les doses d'administration, l'homme du métier pourra également consulter utilement l'article suivant (et les références qui y sont citées) : Daniel DHUMEAUX, La *revue du praticien,* **45,** p. 2519-2522 (1995).

A moins qu'ils ne soient définis d'une autre manière, tous les termes techniques et scientifiques utilisés ici ont la même signification que celle couramment comprise par un spécialiste ordinaire du domaine auquel appartient cette invention.

### Propriétés cliniques des produits de l'invention

### Exemple 1 :

Un groupe de 10 malades atteints d'hépatite C a été traité successivement par du poly(A)-poly(U), puis par de l'interféron.

Le traitement s'est déroulé de la façon suivante :
- dans un premier temps, les malades ont reçu deux fois par semaine durant 24 semaines une dose de 150 mg de poly(A)-poly(U) administrée par voie intraveineuse ;
- à l'issue de ce premier traitement, les malades ont reçu 3 fois par semaine durant 24 semaines une dose de 3 millions d'unités d'interféron administrée par voie intraveineuse.

A l'issue de ce double traitement, on a observé une rémission de la maladie chez 6 patients, une rémission suivie d'une rechute chez un septième patient, tandis que le traitement n'a pas eu d'effet pour 3 patients seulement.

A titre de comparaison, un traitement effectué uniquement avec de l'interféron offre une rémission dans seulement 20 à 30 % des cas (cf. Daniel DHUMEAUX, *La revue du praticien*, **45,** *p.* 2519-2522 (1995)).

### Exemple 2 :

Deux groupes de malades atteints d'hépatite B chronique active, le premier constitué de 42 patients (groupe A), le second de 44 patients (groupe B), reçoivent, à partir de la même date, les traitements suivants :
- les malades du groupe A reçoivent deux fois par semaine durant 24 semaines une dose de 150 mg de poly(A)-poly(U) administrée par voie intraveineuse, puis trois fois par semaine durant les 24 semaines qui suivent une dose de 6 millions d'unités d'IFN-α administrée par voie sous-cutanée ;
- les malades du groupe B ne reçoivent aucun traitement pendant les 24 premières semaines, puis reçoivent trois fois par semaine durant les 24 semaines suivantes une dose de 6 millions d'unités d'IFN-α administrée par voie sous-cutanée.

On détermine la présence d'ADN du virus HBV et la séroconversion HBe 24, 48 et 72 semaines après la date de début du traitement éventuel par du poly(A)-poly(U). Les résultats, exprimés en pourcentage des patients répondant au traitement, sont résumés dans le tableau ci-après :

| Critère | Après 24 semaines | | Après 48 semaines | | Après 72 semaines | |
|---|---|---|---|---|---|---|
| | Groupe A | Groupe B | Groupe A | Groupe B | Groupe A | Groupe B |
| Absence d'ADN du virus HBV ou réduction de plus de 50 % | 40 % | 37 % | 94 % + | 58 % + | 71 % * | 39 % * |
| Séroconversion HBe | 14 % | 7 % | 29 % | 14 % | 33 % ** | 9 % ** |

| | | | | | | |
|---|---|---|---|---|---|---|
| ⁺ p = 0,001 ; | | | | | | |
| * p = 0,02 ; | | | | | | |
| ** p = 0,006 | | | | | | |

Conclusion : chez les patients atteints d'hépatite B chronique active, le prétraitement par le poly(A)-poly(U) avant le traitement par l'IFN-α a eu pour résultat d'accroître le taux de réponse au traitement de 6 mois par l'IFN-α et a diminué le nombre de rechutes après ce traitement.

## Revendications

1. Utilisation d'au moins un ARN double brin (ARNdb) et d'au moins un interféron pour préparer un médicament destiné au traitement d'une hépatite virale, **caractérisée en ce que** l'administration a lieu sous forme d'une association étalée dans le temps et l'administration d'ARNdb a lieu avant celle de l'interféron.

2. Utilisation selon la revendication 1, **caractérisée en ce que** l'ARNdb est l'acide polyadénylique complexé avec l'acide polyuridylique.

3. Utilisation selon la revendication 1, **caractérisée en ce que** l'ARNdb est un complexe de l'acide polyinosinique avec l'acide polycytidylique.

4. Utilisation selon l'une des revendications précédentes, **caractérisée en ce que** l'interféron est choisi parmi les interférons α, β et γ ou les interférons consensus correspondants.

5. Utilisation selon la revendication 4, **caractérisée en ce que** l'interféron est un interféron α (IFN-α).

6. Utilisation selon l'une des revendications 1 à 5, **caractérisée en ce qu'**au moins un autre agent anti-viral est en outre utilisé pour préparer le médicament, ledit autre agent anti-viral étant destiné à être administré en association avec l'ARNdb et l'interféron, l'administration dudit autre agent anti-viral étant de plus antérieure à celle de l'ARNdb.

7. Utilisation selon la revendication 6, **caractérisée en ce que** :
i) un agent anti-viral, en association avec
ii) un ARNdb,
et iii) un interféron éventuellement associé simultanément ou séparément à un agent anti-viral agissant directement sur les virus
sont destinés à être administrés de façon séparée dans le temps et dans l'ordre indiqué ci-dessus.

8. Utilisation selon l'une des revendications 1 à 7, **caractérisée en ce que** l'hépatite virale est une hépatite virale chronique.

9. Utilisation selon l'une des revendications 1 à 7, **caractérisé en ce que** l'hépatite virale est l'hépatite B.

10. Utilisation selon l'une des revendications 1 à 7, **caractérisé en ce que** l'hépatite virale est l'hépatite C.

## Patentansprüche

1. Verwendung mindestens einer doppelstrangigen RNA (dsRNA) und mindestens eines Interferons zur Herstellung eines Arzneimittels für die Behandlung einer viralen Hepatitis, **dadurch gekennzeichnet, dass** die Verabreichung in Form einer zeitlich verteilten Kombination stattfindet und die Verabreichung der dsRNA vor der des Interferons stattfindet.

2. Verwendung nach Anspruch 1, **dadurch gekennzeichnet, dass** die dsRNA mit Polyuridylsäure komplexierte Polyadenylsäure ist.

3. Verwendung nach Anspruch 1, **dadurch gekennzeichnet, dass** die dsRNA ein Komplex von Polyinosinsäure mit Polycytidylsäure ist.

4. Verwendung nach einem der vorhergehenden Ansprüche, **dadurch gekennzeichnet, dass** das Interferon aus den Interferonen α, β und γ oder den entsprechenden Consensus-Interferonen ausgewählt ist.

5. Verwendung nach Anspruch 4, **dadurch gekennzeichnet, dass** das Interferon ein Interferon α (IFN-α) ist.

6. Verwendung nach einem der Ansprüche 1 bis 5, **dadurch gekennzeichnet, dass** für die Herstellung des Arzneimittels außerdem mindestens ein anderes antivirales Mittel verwendet wird, das dazu bestimmt ist, in Kombination mit der dsRNA und dem Interferon verabreicht zu werden, wobei die Verabreichung des anderen antiviralen Mittels außerdem vor der der dsRNA stattfindet.

7. Verwendung nach Anspruch 6, **dadurch gekennzeichnet, dass**
i) ein antivirales Mittel in Kombination mit
ii) einer dsRNA,
und iii) einem Interferon, das ggf. gleichzeitig oder getrennt mit einem direkt auf die Viren einwirkenden antiviralen Mittel kombiniert ist,
dazu bestimmt sind, zeitlich getrennt und in der oben angegebenen Reihenfolge verabreicht zu werden.

8. Verwendung nach einem der Ansprüche 1 bis 7, **dadurch gekennzeichnet, dass** die virale Hepatitis eine chronische virale Hepatitis ist.

9. Verwendung nach einem der Ansprüche 1 bis 7, **dadurch gekennzeichnet, dass** die virale Hepatitis die Hepatitis B ist.

10. Verwendung nach einem der Ansprüche 1 bis 7, **dadurch gekennzeichnet, dass** die virale Hepatitis die Hepatitis C ist.

## Claims

1. Use of at least one double-stranded RNA (dsRNA) and at least one interferon for preparing a medicament intended for the treatment of a viral hepatitis, **characterized in that** the administration takes place in the form of a combination spread over a period of time and the administration of the dsRNA takes place before that of the interferon.

2. Use according to claim 1, **characterized in that** the dsRNA is polyadenylic acid complexed with polyuridylic acid.

3. Use according to claim 1, **characterized in that** the dsRNA is a complex of polyinosinic acid with polycytidylic acid.

4. Use according to one of the preceding claims, **characterized in that** the interferon is chosen from interferons α, β or γ or the corresponding consensus interferons.

5. Use according to claim 4, **characterized in that** the interferon is an interferon-α (INF-α).

6. Product according to one of claims 1 to 5, **characterized in that** at least one other antiviral agent is moreover used for preparing the medicament, said other antiviral agent being intended to be administered in combination with the dsRNA and the interferon, the administration of said other antiviral agent being moreover prior to that of the dsRNA.

7. Use according to claim 6, **characterized in that**:
i) an antiviral agent, in combination with
ii) a dsRNA,
and iii) an interferon optionally combined simultaneously or separately with an antiviral agent acting directly on the viruses.
are intended to be administered separately over time and in the order indicated above.

8. Use according to one of claims 1 to 7, **characterized in that** the viral hepatitis is a chronic viral hepatitis

9. Use according to one of claims 1 to 7, **characterized in that** the viral hepatitis is hepatitis B.

10. Use according to one of claims 1 to 7, **characterized in that** the viral hepatitis is a hepatitis C.
